# EUROPÄISCHE PATENTSCHRIFT

(11) **EP 2 473 594 B1**
(45) Veröffentlichungstag und Bekanntmachung des Hinweises auf die Patenterteilung: **05.03.2014**
(21) Anmeldenummer: 10760943.0
(22) Anmeldetag: 03.09.2010
(51) Int. Cl.: C12M 1/40, C12M 3/02

(54) **VORRICHTUNG UND VERFAHREN ZUR EXPANSION, ERNTE UND DIFFERENZIERUNG VON STAMMZELLEN**
DEVICE AND METHOD FOR THE EXPANSION, HARVESTING AND DIFFERENTIATION OF STEM CELLS
DISPOSITIF ET PROCÉDÉ POUR L'EXPANSION, LA RÉCOLTE ET LA DIFFÉRENCIATION DE CELLULES SOUCHES

(30) Priorität: 04.09.2009 EP 09169558
(43) Veröffentlichungstag der Anmeldung: 11.07.2012
(73) Patentinhaber: Technische Hochschule Mittelhessen, 35390 Giessen (DE)
(72) Erfinder: CZERMAK, Peter, 35576 Wetzlar (DE); FREIMARK, Denise, 35398 Giessen (DE); GRACE, Pablo, Pino, 35392 Giessen (DE); JUSTICE, Christiane, 35398 Giessen (DE); WEBER, Christian, 35102 Damm (DE)
(74) Vertreter: Patentanwälte Olbricht Buchhold Keulertz
(86) Internationale Anmeldenummer: PCT/EP2010/062952
(87) Internationale Veröffentlichungsnummer: WO 2011/026939

(56) Entgegenhaltungen:
- EP-A1- 0 953 633
- WO-A1-2008/101215
- WO-A2-03/074668
- WO-A2-2007/022043
- US-A- 5 811 301
- US-A1- 2005 213 374
- US-A1- 2006 240 550
- US-A1- 2008 305 146

## Beschreibung

Die vorliegende Erfindung betrifft ein Verfahren zur Expansion, Ernte und Differenzierung von Stammzellen z.B. humanen mesenchymalen Stammzellen für die Herstellung von zelltherapeutischen Implantanten unter Nutzung von Festbettreaktoren und der Impedanzspektroskopie und eine Vorrichtung durch Durchführung des Verfahrens.

Mit der Zelltherapie werden Patienten behandelt, bei denen lebenswichtige Organe funktionsunfähig sowie ganz oder teilweise zerstört sind, wie z.B. bei Diabetes mellitus oder Schlaganfall. Die Basis vieler Implantate sind genetisch veränderte mesenchymale Stammzellen (hMSC), welche zuvor unter GMP-Bedingungen expandiert und mit hoher Vitalität bei gleichbleibend hoher Qualität geerntet werden müssen. Durch das Implantieren von Stammzellen werden Beschwerden, die das geschädigte Organ verursacht, gelindert. Die z.B. genmodifizierten oder präparierten Stammzellen werden anschließend häufig mittels semipermeabler und biokompatibler Materialien wie z. B. Alginat verkapselt, wodurch sie physikalisch vor dem Immunsystem des Empfängers geschützt werden. Auf diese Weise kann auch körperfremdes (allogenes) Zellmaterial eingesetzt werden. Dabei können Nährstoffe, Stoffwechselprodukte und therapeutische Moleküle die Membran passieren. Bei herkömmlichen medikamentösen Therapien wird der Wirkstoff stets in höherer Konzentration als notwendig verabreicht, um die Blut-Hirn-Schranke passieren zu können. Die Überrnedikation kann zu Nebenwirkungen führen. In diesem Punkt zeigen Zellimplantate einen wesentlichen Vorteil. Der Wirkstoff kann direkt an dem Ort, wo er benötig wird, abgegeben werden. Für die Herstellung solcher stammzellbasierter Implantate müssen die Zellen jedoch unbedingt in ausreichender Zahl vorhanden sein.

Der Bedarf an Stammzellen für die Zelltherapie ist steigend, wodurch die Notwendigkeit für die Etablierung einer schnellen und zuverlässigen Produktion der Zellen insbesondere unter GMP-Bedingungen erforderlich wird.

Die Massenkultivierung von adhärenten Stammzellen ist mit verschiedenen Problemen verbunden und derzeit ist es nicht möglich, eine direkte Kontrolle des Differenzierungsstatus und der Vitalität von Zellen in einem Bioreaktor zu erzielen und die Kultivierung dieser Stammzellen zu automatisieren.

Im Stand der Technik werden Stammzellen adhärent auf zweidimensionalen Flächen kultiviert. Systeme wie Well-Platten, T-Flaschen und gasdurchlässige Blutbeutel werden aufgrund ihrer Einfachheit, leichten Handhabung und geringen Kosten für das Screening, die Entwicklung von Geweben und die einfache Stammzellhaltung eingesetzt. Bei diesen Systemen fehlt jedoch die Möglichkeit des online-Monitoring. Weiterhin sind sie bedingt durch das Flächen-Volumen-Verhältnis schwer skalierbar und somit für die Bioproduktion ungeeignet.

Nachteilig an statischen Bioreaktoren, bei denen der Transport von Nährstoffe, Zellen, Metaboliten, Sauerstoff und anderen Stoffen nur per Diffusion erfolgt, ist, dass sie nur für geringe Zelldichten und niedrige Ausbeuten an Stammzellen geeignet sind, zudem sind diese Systeme inhomogen.

Die WO 03/074668 A2 schlägt eine Vorrichtung und ein Verfahren zur Vermehrung und Differenzierung von homozygoten Stammzellen vor, das Reaktorensemble einen Festbettreaktor und ein damit verbundenes Konditionierungsgefäß umfasst. Die Gefäße sind über Leitungen und Pumpen miteinander verbunden. Die Nährstoffkonzentration, einige Zellparameter sowie Kulturmesswerte werden von Sensoren erfasst und überwacht. Eine derartige Vorrichtung und ein solches Verfahren sind jedoch nicht zur Massenherstellung von Stammzellen geeignet.

### Aufgabe

Aufgabe der Erfindung ist es die Nachteile im Stand er Technik zu beseitigen und ein Verfahren und eine Vorrichtung zur Durchführung des Verfahrens bereitzustellen, das zur Massenherstellung von Stammzellen geeignet ist.

### Lösung

Die Aufgabe wird gemäß Anspruch 2 gelöst, indem ein Verfahren zur Expansion, Ernte und Differenzierung von Stammzellen bereitgestellt wird, das eine direkte und automatische Kontrolle des Differenzierungsstatus, der Vitalität und der Ernte von Stammzellen erlaubt und zur Massenherstellung von Stammzellen geeignet ist. Weiterhin wird die Aufgabe gemäß Anspruch 1 gelöst und eine Vorrichtung zur Durchführung des Verfahrens bereitgestellt.

Vorteilhaft ist, dass erstmals die Expansion, Ernte und Differenzierung von großen Mengen an Stammzellen z.B. als Implantate für die Zelltherapie in einem vollständig automatisierten Bioreaktorsystem unter sterilen Bedingungen möglich ist, wobei das Verfahren zur automatisierten Durchführung die Impedanzspektroskopie nutzt.

Dabei wird der Bedarf von Nährstoffen, Medium, Metaboliten, Sauerstoff und anderen Stoffen in den Bioreaktor gemessen und deren Transport in den Bioreaktor kontrolliert und ermöglicht. Dieses Verfahren wird als batch-Verfahren oder kontinuierlich durchgeführt, wobei 10fach höhere Zelldichten, im Vergleich zu statischen Methoden erreicht werden. Das scale-up dieser Prozesse ist auf Grund des verbesserten und automatisierten Stofftransportesrelativ einfach.

Stammzellen sind erfindungsgemäß embryonale oder adulte Stammzellen, z.B. humane mesenchymale Stammzellen, wie die adhärente humane mesenchymale Stammzelllinie hMSC-TERT, die natürlichen Ursprungs ist oder gentechnisch verändert wurde. hMSC-TERT sind genetisch modifizierte humane mesenchymale Stammzellen, in welchen die Telomerase Reverse Transkriptase (TERT) transfiziert wurde. Diese erlaubt eine Erhöhung der Generationszahl, da die Telomere nicht bei jeder Zellteilung verkürzt werden. Für Zelltherapie werden alternativ noch Gene für therapeutische Proteine in die Zellen kloniert.

Die Herstellung der Stammzellen erfolgt erfindungsgemäß in Festbettreaktoren oder Einweg-Festbettreaktoren, wobei die Prozesskontrolle vorzugsweise online unter Nutzung der Impedanzspektroskopie erfolgt. Dies ermöglicht es, die Zellzahl der Stammzellen, deren Vitalität und Differenzierungsstatus zu kontrollieren und den gesamten Kultivierungsverlauf so zu automatisieren, dass ein exakter Erntezeitpunkt festgelegbar ist.
Erfindungsgemäß sind Einwegreaktoren und Einweg-Festbettreaktoren Vorrichtungen, die zur Kultivierung (Suspensions- und adhärente Kulturen) von scher-empfindlichen Zellen (tierische und pflanzliche Zellen, Stammzellen) für die Produktion von Pharmazeutika (z.B. Impfstoffe, Antikörper, Zellen), für Hochzelldichtefermentationen (bis zu 500L Reaktionsvolumen), ebenso wie für Inokula, Medienherstellung und Mischprozesse geeignet sind. Einwegreaktorsysteme werden in drei Kategorien eingeteilt: bag- Reaktoren, small-scale Systeme mit Modifikationen (spinner-Flaschen, T-Flaschen, roller-bottles) sowie Hohlfaserreaktoren.
Die Einwegsysteme weisen mehrere Vorteile auf. So ist z.B. keine Autoklavierung (SIP= sterilization in place) und Reinigung (CIP= Cleaning in place) notwendig, wodurch eine Reduzierung der Prozesszeiten folgt, da die Leerzeiten zwischen Chargen entfallen, zudem wird das Kreuzkontaminationsrisiko reduziert. Weiterhin ist der Qualifizierungs- und Validierungsaufwand eines Prozesses reduziert. Diese Faktoren führen zu einer Kostenreduktion von bis zu 50% .

Die Vorrichtung zur Expansion, Ernte und Differenzierung von Stammzellen umfasst einen Bioreaktor der einen Festbettreaktor (R1) mit einem daran gekoppelten Konditionierungsgefäß (R2) aufweist, wobei Festbettreaktor (R1) und das Konditionierungsgefäß (R2) über Leitungen mit Pumpen (P1, P2) gekoppelt sind und eine Prozesssteuerung vorhanden ist, die Messwerte von Kontrolleinheiten (S1, S2, S3), die mit einer Impedanzsonde erzeugt werden, entgegen nimmt und daraufhin Ventile (V1, V2, V3, V4, V4, V6, V7, V8, V9) steuert, sodass dem Festbettreaktor (R1) aus Gefäßen (G1, G2, G3, G4) Nährstoffe zugeführt werden und die Expansion, Ernte und Differenzierung der Stammzellen automatisch und steril erfolgt.

Die erfindungsgemäße Vorrichtung weist insbesondere einen Einwegreaktor oder einen Einweg-Festbettreaktor (R1) auf. Dieser Reaktor ist mit einem Konditionierungsgefäß (R2) gekoppelt. Im Konditionierungsgefäß befindet sich das für Stammzellen geeignete Kultivierungsmedium, wo es mit Gasen z.B. Sauerstoff angereicht wird. Am Zu- und Ablauf des Reaktors (R1) ist mindestens eine Kontrolleinheit oder mindestens ein Sensor (S1, S2, S3, S4, S5) z.B. ein optischer Sauerstoffsensor angebracht, dessen Messwerte eine Aussage über das Wachstum der Zellen erlaubt. Zu- und Abläufe werden durch Ventile (V1- V9) und ein Prozessleitsystem gesteuert.
Dieses Prozessleitsystem wird vorzugsweise mit einer Software z.B. Labvision (Hightec Zang, Herzogenrath, D.) gesteuert.
Die mindestens eine Kontrolleinheit oder der mindestens eine Sensor (S1, S2, S3, S4, S5) ist z.B. ein optischer oder faseroptischer Sauerstoffsensor, der die Sauerstoffkonzentration online bestimmt z.B. ein Sensor von PreSens, Regensburg.

Die Kultivierung der Stammzellen erfolgt wie im Stand der Technik bekannt ist auf geeigneten Zellträgern, z.B. aus Borsilikat oder Dextran oder anderen für die Zellkultur geeigneten Materialien. Auf diesen Trägern haften sich die Stammzellen z.B. die adhärente humane mesenchymale Stammzelllinie (hMSC-TERT) gut an und expandieren.

Für Abfall, Enzym- und Pufferlösung ist das Bioreaktorsystem mit Gefäßen (G1 bis G5) gekoppelt. Ein Vorratsgefäß (G5) ist durch eine separate Leitung direkt mit dem Konditionierungsgefäß (R2) verbunden. Dieses Vorratsgefäß (G5) beinhaltet ebenfalls Medium, welches im Falle eines Medienwechsels direkt in das Konditionierungsgefäß (G5) überführt werden kann. Dieser Vorgang wird ebenfalls durch das Prozessleitsystem gesteuert.
Die gesamte Vorrichtung ermöglicht eine optimale Expansion und Ernte von Stammzellen, so dass die Stammzellen anschließend in Alginat verkapselt werden und eine Differenzierung dieser verkapselten Stammzellen erfolgt.

Das erfindungsgemäße Bioreaktorsystem weist eine erhöhte Prozesssicherheit auf und ermöglicht auch den automatischen Mediumswechsel und die automatische Ernte der Stammzellen. Dabei wird die Expansion der Stammzellen ständig anhand des Glukoseverbrauchs überwacht, wobei die optimalen Zeitpunkte für die Einleitung eines Mediumwechsels bzw. der Ernteprozedur bestimmt werden. Erstmals ist eine optimale Kontrolle der gesamten Produktionsprozesse der Stammzellen möglich, indem die Systemüberwachung vorzugsweise durch Impedanzspektroskopie erfolgt, welche die elektrischen Eigenschaften der Zellen ausnutzt.

Der Einsatz der Impedanzmessung führt zu einer Vollautomatisierung des Systems und zu einer Erhöhung der Prozesssicherheit durch permanente online-Überwachung der Lebendzellmasse und somit Steuerung des Expansions- und Ernteprozesses. Dies ist für die Durchführung eines GMP-gerechten Prozess zur Massenproduktion von Stammzellen ein großer Fortschritt.

### Aufbau des Bioreaktorsystem

Das Bioreaktorsystem besteht aus einem bekannten handelsüblichen Bioreaktor, beispielsweise einem Festbett oder einem Wirbelbett, gekoppelt an einem Konditionierungsgefäß z.B. ein Rührkesselreaktor und eine Begasungseinheit in Form eines Membranmoduls. Durch die Integration einer Parameterkontrolleinheit zur Darstellung unterschiedlicher Niveaus eines oder mehrerer Prozessparameter während des Herstellungsprozesses werden bei der vorliegenden Erfindung das Animpfen, die Adhäsion und der Status der Adhärentekultur sowie die Vermehrung der Produktzelllinie und die Ernte überwacht und gesteuert.

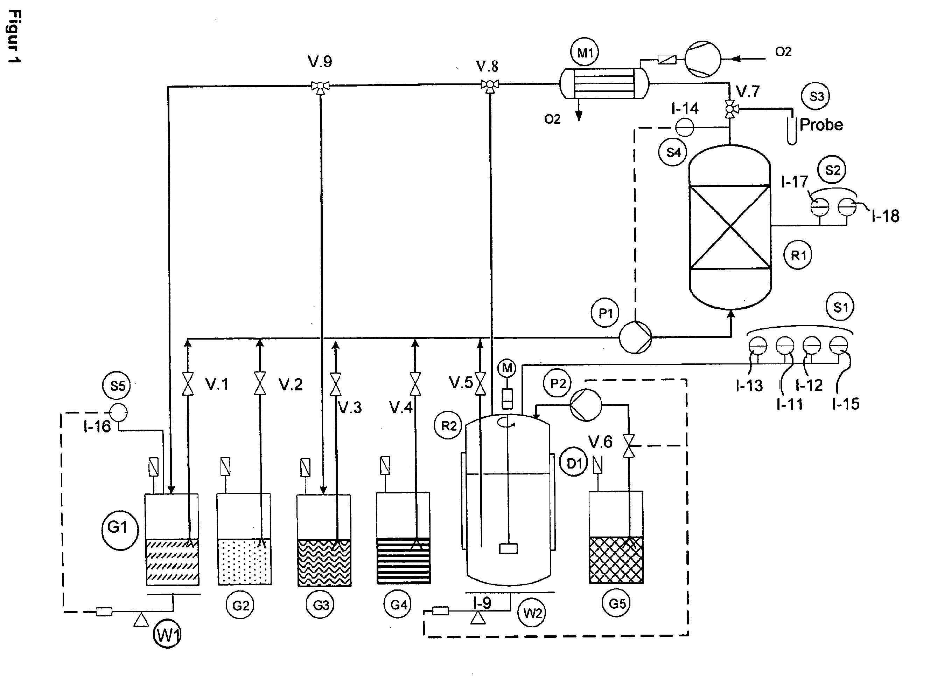

Figur 1 zeigt die schematische Darstellung eines erfindungsgemäßen Ausführungsbeispieles.
Der Bioreaktor z.B. Festbettreaktor (R1) ist mit einem Konditionierungsgefäß z.B. Rührkesselreaktor (R2) über eine Leitung mit einer Pumpe z.B. Schlauchpumpe (P1) gekoppelt. Um die Adhärentekultur ausreichend mit Nährstoffen z.B. Glucose zu versorgen, wird anhand des gemessenen Werts am Messort der Kontrolleinheit (S3) die Drehzahl am (P1) und somit die Strömungsgeschwindigkeit im (R1) erhöht. Alternativ wird für den Nährstoff Sauerstoff ein Begasungssystem wie z.B. Membranmodul (M1) am Ausgang vom Reaktor (R2) angebracht. Alternativ wird direkt im Reaktor (R2) das Medium mit Sauerstoff angereicht. Das Medium wird mittels einer Wärmevorrichtung zum Beispiel ein Doppelmantel (D1) am Reaktor (R2) zu der gewünschte Temperatur erwärmt. Wichtige Prozessparameter werden an der Kontrolleinheiten (S1), (S2), gemessen z.B. pH-Wert, Druck, Temperatur, Sauerstoff und Biomasse. Für die offline Überwachung wie z.B. die Glukosebestimmung ist am (S3) ein Probenahmesystem angebracht z.B. eine S-Monovette®. Während der Kultivierung der Adhärente Zelllinie müssen mehrere Medienwechsel durchgeführt werden, hierfür werden die Ventile (V.8) und (V.9) so geschaltet dass das zu ersetzende Medium in einem Abfallgefäß (G3) geführt wird. Die automatische Mediumnachfüllung im Reaktor (R2) wird anhand der Volumenänderung im Reaktor eingeleitet. Für die Erfassung der Volumenänderung wird ein Meßsystem verwendet z.B. eine Waage (W2) welche bei einem minimalen Wert das Ventil (V.6) und die Pumpe (P2) öffnet bzw. einschaltet, beim Erreichen eines vorgegebenen Volumens im Reaktor (R2) wird die Pumpe (P2) und das Ventil (V.6) ausgeschaltet bzw. geschlossen. Um die Beendigung der Kultivierung zu steuern, wird im Reaktor (R1) eine Online Überwachung am (S2) mit einer Impedanzsonde angebracht. Anhang dieser Messung werden die weiteren Schritten angeleitet. Beim Erreichen einer bestimmten Endzelldichte wird das System entleert indem das Kultivierungsmedium ins Abfallgefäß (G3) geleitet wird. Nach der Entleerung wird die kultivierte Zelllinie mit einer Pufferlösung im Gefäß (G4) z.B. PBS gespült. Nach dem Spülen werden die an den Trägern haftenden Zellen geerntet. Für die Ernte wird das im Gefäß (G2) befindliche Enzym in den Reaktor (R1) geführt.
Nach dem Ablösen der Zellen (automatische Überwachung durch die Impedanzsonde) wird der Reaktor (R1) mit Medium aus dem Konditionierungsgefäß (R2) durchströmt, die Ventile (V.8) und (V.9) werden so geschaltet, dass die Zellsuspension ins Auffanggefäß geleitet wird. Im Auffanggefäß befindet sich eine Impedanzsonde, die den Verlauf der Ernte beobachtet. Um die zeitliche Volumenänderung im Auffanggefäß zu erfassen, wird eine Waage (W1) angebracht, mit der, in Kombination mit der Impedanzsonde, die zunehmende Zellkonzentration bestimmt wird. Bleibt die Zellkonzentration im Auffanggefäß stabil, wird der Prozess beendet.
Die Anbringung der Impedanzsonde erfolgt wahlweise an verschiedenen Positionen am erfindungsgemäßen Bioreaktorsystem, wobei die Höhe der Anbringung variieren kann. Alternativ werden auch mehr als eine Sonde angebracht.

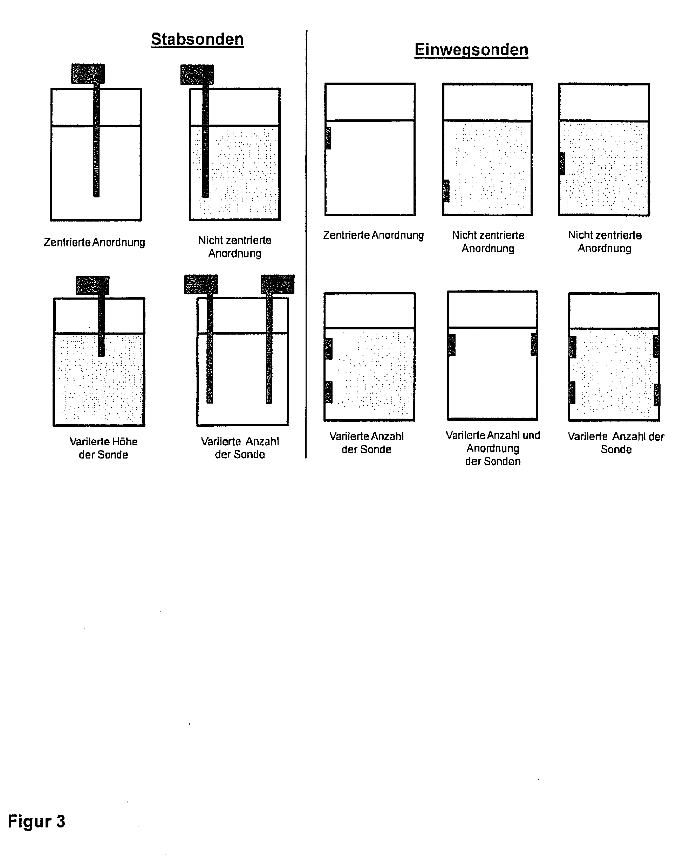

Figur 3 zeigt die zentrierten oder nicht-zentrierten Positionen für die Anbringung der Impedanzsonde z.B. am axial durchströmten zylindrischen Festbettreaktor, wobei die Darstellung die Sondenformen: runde Stabsonde mit verschiedenen Durchmessern oder flache Form als Einwegartikel berücksichtigt.
Bei der Verwendung runder Stabsonden ist zu beachten, dass die Stabsonde über den Reaktordeckel in den Reaktor eingebracht wird, wobei hier die Sondeneinbringung über geeignete Gewinde gemäß steriltechnischen Anforderungen erfolgt. Der Abstand der Sonde zur Mittelachse des Reaktors kann variieren, ebenso wie die Höhe des Sondenkopfes im Verhältnis zur Reaktorhöhe. Einwegsonden werden im *"Bag"* eingeschweißt und die Kabelanschlüsse zeigen nach außen. Dabei ist die Sondenanbringung an der Reaktorwand auf verschiedenen Höhen möglich. Es werden alternativ mehrere Sonden in unterschiedlicher Höhe und in unterschiedlichem Abstand zueinander angebracht. Die Anzahl der Sonden ist dabei variabel. Figur 3 zeigt bevorzugte Anbringungsvarianten der Sonden.

Die Sondenanbringungsmöglichkeiten gelten für den Wirbelbettreaktor als Alternative zum axial durchströmten Festbettreaktor analog. Für das Erntegefäß gelten analoge Sensoreinbindungsmöglichkeiten wie für die Stabsonden im zylindrischen axial durchströmten Festbettreaktor. Hier ist zu beachten, dass keine zentrierte Anbringung möglich ist, da die Rühreinheit dort eingesetzt ist. Die Sonden benötigen einen ausreichenden Abstand zur Rührerwelle und den Rühreinheiten. Der Deckel des Reaktors ist abhängig von der Anbringung der Sonden mit den passenden Gewinden an den gewünschten Positionen.
Für die Differenzierung im Reaktor sind die Möglichkeiten die Sondenanbringung analog denen für den Animpf- und Expansionsprozess.

Erfindungsgemäß wird ein Verfahren zur Expansion, Ernte und Differenzierung von Stammzellen bereitgestellt, das die Schritte umfasst:
1. Bereitstellen eines Bioreaktor mit geeignetem Medium, animpfen mit einer Starterkultur aus Stammzellen
2. Messung der Sauerstoff-, Glukose- und/oder Laktatkonzentration, der Zelldichte, der Zellzahl, der Zellgrößenverteilung im Bioreaktor durch Impedanzspektroskopie
3. Vergleich der Messwerte aus Schritt 2 mit Referenzwerten
4. Automatische Zufuhr von Nährstoffen oder Ernte der Stammzellen durch Entleerung des Bioreaktor und Auffangen der Stammzellen
5. Differenzierung des Stammzellen

Vorzugsweise erfolgt die Messung der Sauerstoffkonzentration online. Vorzugsweise erfolgt die Messung der Glukose- und Laktatkonzentration durch Probennahme.

Das Kultivierungsende und damit das Einleiten des Ernteprozesses wird durch die ermitteltezellkonzentration aus den Messungen im Medium bestimmt. Dies ist möglich, da nach Bestimmung des spezifischen Glukoseverbrauchs über den Glukoseverbrauch indirekt die Zellzahl ermittelt wird. Der Volumenstrom wird durch den Sauerstoffgehalt am Reaktorausgang gesteuert.
Die Vitalität der Zellen wird durch Probenahme und Zellzahlbestimmung mittels Trypan-Blau-Ausschluss-Methode ermittelt. Das Ablösen der Zellen von den Trägern bei der Ernte und für die Zellzahlbestimmung erfolgt mittels Trypsin/Accutase-Gem isch.
Die Zellzahlbestimmung, die Bestimmung der Zellkonzentration, ebenso Zellgrößenverteilung der Zellen während des Expansionsprozesses erfolgt mittels der Impedanzspektroskopie online.

### Bestimmung von Zellkonzentration und Zellgrößenverteilung

Die Zellzahlbestimmung erfolgt mittels des bekannten Trypanblau-Membran-Integritätstest. Dabei werden ausschließlich tote Zellen gefärbt. Die Zellzahl wird durch Auszählen im Hämozytometer bestimmt.
Eine ähnliche Methode ist das Auszählen der Zellen nach Anfärbung des Zellkerns durch Kristallviolett. Bei beiden Methoden ist das Ablösen von adhärenten Zellen mit Trypsin erforderlich. Eine andere Methode ist die Messung des DNA-Gehaltes, welcher einen Rückschluss auf die Zellzahl erlaubt, da jede Zelle eine definierte DNA-Menge enthält. Hier wird nur die Gesamtzellzahl bestimmt und es ist kein Rückschluss auf die Lebendzellzahl möglich. Alternativ gibt es eine kolorimetrische Quantifizierung der Zellproliferation und -vitalität. Dieser Nachweis basiert auf der Spaltung des Tetrazoliumsalzes WST1 *(watersoluble tetrazolium*) durch mitochondriale Dehydrogenasen in lebenden Zellen, wodurch ein gefärbtes Formazan entsteht. Eine direkte Methode Zellzahlbestimmung ist der Multi-Tox-Test (Promega, Mannheim, D.). Zur Bestimmung der Lebendzellzahl wird bei diesem Test die Enzymaktivität von ausschließlich in lebenden Zellen vorhandenen Proteasen fluorometrisch detektiert.
Weiterhin sind teilautomatische Systeme wie der Cedex von Innovatis (Bielefeld, D.) und das ViCell von Beckman-Coulter (Krefeld, D.) bekannte offline-Systeme. Beide Systeme basieren auf der Zellfärbung mit Trypan-Blau zur Unterscheidung lebender und toter Zellen.

Die Messung zur Zellgrößenverteilung adhärenter Zellen erfolgt durch Analyse des zellspezifischen Proteingehaltes z.B. mittels eines BCA-Assays. Der spezifische Zellproteingehalt ist linear abhängig vom Zellvolumen. Diese Methode erfordert wiederum eine Zellzahlbestimmung und erlaubt nur eine indirekte Bestimmung der Zellgröße.

### Bestimmung von Glukose

Die Sicherung der Glukoseversorgung der Zellen ist essentiell für deren Wachstum. Die meisten bekannten Glukosemessungen erfolgen amperometrisch oder fluoreszenzbasiert. Bei amperometrischen Messmethoden werden Glukoseoxidasen eingesetzt, welche Glukose zu Lakton umsetzen, wobei Elektroden frei werden. Bei dieser Reaktion wird zelltoxisches Wasserstoffperoxid freigesetzt. Ein Produktbeispiel ist der Glukose Analysator 2 (Beckmann), ebenso gibt es Produkte der Firma YSI.
Da der Verbrauch der Glukose während des Kultivierungsprozess nicht nur ein Marker für den Zeitpunkt des Medienwechsels sondern auch ein zusätzliches Indiz für die Zellkonzentration im System ist, erfolgt erfindungsgemäß die Messung der Glukosekonzentration im Reaktor online und automatisch. Dazu wird ein Einweg-online-Glukosesensor z.B. von der Firma C-CIT *Center for Chemical Information Technology* (Wädenswill, CH) verwendet. Dieser Sensor weist eine lineare Konzentrationsabhängigkeit für den Bereich von 2-6mM Glukose auf, ist durch γ-Bestrahlung sterilisierbar und sowohl für Schnelltests (Einfachmessungen) als auch für Zellkulturbeobachtung über zwei Wochen geeignet. Die Korrelation des Messergebnisses erfolgt durch Referenzmessungen z.B. mit HPLC. Nachteilig ist, dass bei der enzymatischen Reaktion während der Messung Wasserstoffperoxid freigesetzt wird, welches die Zellen schädigt (Hofbildung). Diese Hofbildung wird aber durch Ergänzung einer Enzympaste mit Peroxidase verhindert werden.

### Online-Monitoring

Für die Prozesssteuerung ist die Überwachung von Parametern wie O₂, CO₂, pH, Temperatur, Glukose-, Laktat-, sowie die Zellkonzentration entscheidend. Ausgenommen der Glukose- und Laktatkonzentration ist für die genannten Parameter entsprechende online-Analytik auf dem Markt verfügbar und etabliert. Beispiele hierfür miniaturisierte Einmal-Sensoren auf elektrochemischer Basis zur Messung von O₂ und pH von der Fa. Applisens einem Tochterunternehmen der Fa. Applikon oder miniaturisierte Sensoren auf optischer Basis (Fluoreszenz) von YSI, PreSens. Solche Sauerstoffsensoren der Firma PreSens sind am IBPT vorhanden und werden erfindungsgemäß eingesetzt.

### Bestimmung von Zellkonzentration und Zellgrößenverteilung

Die Zellkonzentration und die Zellgrößenverteilung sind wichtige Parameter für biopharmazeutische Prozesse. Neben Trübungsmessverfahren werden für das online-Monitoring auch bildgebende Verfahren für die Bestimmung der Prozessparameter eingesetzt. Ein Beispiel ist die In-situ-Mikroskopie (ISM), welche die Bestimmung direkter und indirekter Zellparameter in Echtzeit und ohne Probenahme erlaubt. Bekannte Geräte zur Zellzahlbestimmung sind das PVM (Particle Vision and Measurement) von Mettler Toledo/Lasentec und das ISM von Sartorius. Eine bevorzugte Möglichkeit der online-Zellzahlbestimmung ist die Verwendung der Impedanzspektroskopie.

### Bestimmung von Sauerstoff

Die Bestimmung des Sauerstoffpartialdruckes im Medium wird zumeist mittels Clark-Sonden bestimmt. Auch bekannt ist der Einsatz faseroptische Sauerstoffsensoren, basierend auf Fluoreszenzquenching. Diese Sensoren sind z.B. auch als Einwegsensoren (PreSens, YSI, Applisens) erhältlich.

### Impedanzspektroskopie

Für die pharmazeutischen Industrie ist eine hochentwickelte online Prozessüberwachung wichtig.
Für die Prozessüberwachung sind *in-situ* Messungen ideal, wobei geeignete Sensoren biokompatibel und aus inerten Materialien gefertigt sein sollten. Weiterhin müssen diese Sensoren ohne Kalibrierung einen Prozess kontinuierlich über einen Zeitraum von bis zu vier Wochen zuverlässig überwachen. Für die Bestimmung des Sauerstoff- und Kohlendioxidpartialdruckes, von pH und Temperatur sind diese Voraussetzung der Sensorik bereits gegeben. Diese Prozessparameter werden ebenso wie Glukose- und Laktatkonzentrationen üblicherweise für die Prozessbeschreibung verwendet.

Die dielektrische Spektroskopie, auch Impedanzspektroskopie genannt, nutzt die elektrischen Eigenschaften der Zellen, welche einem elektrischen Feld ausgesetzt werden und wird so erfindungsgemäß zur online-Zellzahlbestimmung verwendet. Dabei funktionieren Zellen mit einer intakten Plasmamembran wie ein Kondensator, da die nicht-leitende Eigenschaft der Zellmembran zu einer Polarisierung der Zelle führt. Während einer Batch-Kultur verändert sich die spezifische Zellkapazität um bis zu 45%, wobei der höchste Wert bei der höchsten Zellzahl erreicht wird. Tote und teillysierte Zellen, sowie Zellbruchstücke, Gasblasen und andere Medienkomponenten werden von der Sensorik nicht erfasst.

Das Messergebnis ist die resultierende Kapazität bzw. die dielektrische Permeativität in pF (pico-Farad), welche vom Zelltyp, der Zellgröße und daraus resultierend dem Zellvolumen abhängig, und in einem bestimmten Messbereich linear zur Zellkonzentration ist. Die Sonde baut ein 20-25mm großes Frequenzfeld um den Sondenkopf auf. Im niedrigen Frequenzbereich (0.1-10Mhz), im Bereich der zelltypischen kritischen Frequenz (fc), ist die Auslenkung der Amplitude höher, die Ionen akkumulieren an der Zellmembran.

Die Ladungsakkumulation polarisierter Zellen ist abhängig von der Zelldichte. Wird bei einer festen Frequenz (kritische Frequenz) gemessen, so ist die Permeativität abhängig von Zellgröße und Status. Bei niedrigeren Frequenzen, wenn die Zelle vollkommen polarisiert ist, steigt der Wert der Kapazität mit steigender Ladungsverdichtung nicht weiter an. Die Steigerung der Permeativität von hohen zu niedrigen Frequenzen folgt einer sigmoidalen Kurve und ist als ß-Verteilung bekannt. Diese Methode liefert keine Signale über die intrazellulären Komponenten. Daher sind Aussagen über metabolische Aktivitäten nur schwer möglich.

Vorteile dieser Methode gegenüber z.B. Trübungsmessungen sind die geringe Anfälligkeit gegenüber nicht zellulären Partikeln oder *fouling* der Sonde.

Üblicherweise ist die Sonde eines dieletrischen Spektrometers mit vier Elektroden besetzt und ist passkonform mit Standardfermentern sowie dampfsterilisierbar. Die Sondenintegration und Prozessführung ist auch für große Volumina. (scale-up) geeignet.
Das Produktionssystem ist nicht nur für mesenchymale Stammzellen, sondern für verschiedenste adhärent wachsenden Systeme geeignet. Das System erlaubt eine Reduktion der *"time to market"* durch vereinfachte Prozessentwicklung mit neuen Zellinien.

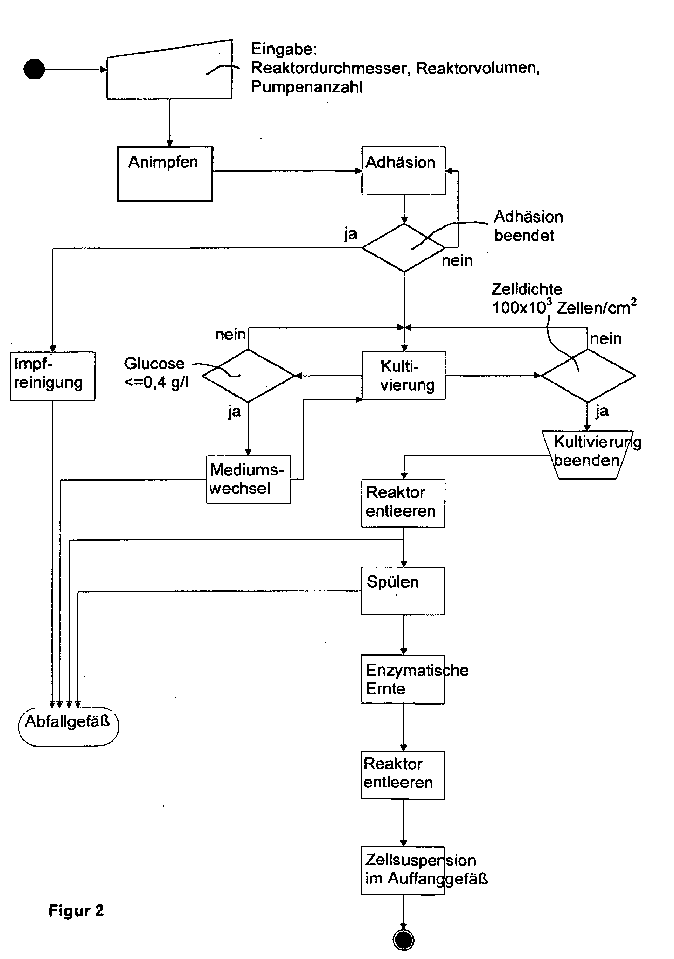

Figur 2 zeigt als Ablaufsdiagramm das erfindungsgemäße Verfahren.

Der Herstellungsprozess ist in drei Bereiche zu untergliedern: Immobilisierung, Expansion und Ernte. Für die Expansion sind die Überwachung des Zellwachstums und die Bestimmung der maximalen Zelldichte vor Einleiten der Ernte wichtig. Die Zellkonzentration, ebenso wie die Zellgrößenverteilung der Zellen wird während des Expansionsprozesses mittels der Impedanzspektroskopie online bestimmt. Weiterhin wird die Glukosekonzentration offline und online bestimmt. Die offline-Messmethode erfolgt mit Probenahme unter sterilen Bedingungen.

Erfindungsgemäß wird ein Verfahren zur Expansion, Ernte und Differenzierung von Stammzellen bereitgestellt, das die Schritte umfasst:
1. Bereitstellen eines Bioreaktor mit geeignetem Medium, animpfen mit einer Starterkultur aus Stammzellen
2. Messung der Sauerstoff-, Glukose- und/oder Laktatkonzentration, der Zelldichte, der Zellzahl, der Zellgrößenverteilung im Bioreaktor durch Impedanzspektroskopie
3. Vergleich der Messwerte aus Schritt 2 mit Referenzwerten
4. Automatische Zufuhr von Nährstoffen oder Ernte der Stammzellen durch Entleerung des Bioreaktor und Auffangen der Stammzellen
5. Differenzierung des Stammzellen

Das Animpfen mit einer Starterkultur aus Stammzellen erfolgt manuell direkt in den Reaktor oder als Inokulum in Gefäß (G1), welches es dem Reaktor (R1) automatisch zuführt. Mit der Impedanzsonde wird die genaue Zelldicht beim Animpfen beobachtet und dokumentiert.

Die Expansion der Stammzellen aus der Starterkultur wird online durch Messung der Glukosekonzentration anhand einer der Prozesssteuerung hinterlegten Minimalkonzentration überwacht und bei Bedarf ein Mediumwechsel automatisch eingeleitet und durchgeführt. Dies führt zu einer Vollautomatisierung des Systems und zu einer Erhöhung der Prozesssicherheit durch permanente online-Überwachung der Lebendzellmasse und der Nährstoffversorgung (Glukose). Alternativ wird der Mediumwechsel auch manuell eingeleitet.

Die Ernte der Stammzellen erfolgt bei niedriger Durchströmungsgeschindigkeit und längerer Zeit oder alternativ bei hoher Durchströmungsgeschindigkeit und kurzer Zeit oderalternativ durch pulsierende Strömungstöße im Reaktor.

### Anwendungsbeispiel einer Sonde

Ein Beispiel einer solchen online- Messsonde ist die Impedanzspektroskopie. Mittels dieser Sonde wird die Kapazität der Biomasse bestimmt. Damit ist das Animpfverfahren, die Expansion der Stammzellen, sowie das Ernteverfahren der Stammzellen beobachtbar und steuerbar. Alternativ ist auch der Differenzierungsprozess der Zellen beobachtbar und steuerbar, da sich hier die Membraneigenschaften verändern, welche mittels der Impedanzmessung detektierbar sind.

### Animpfprozess

Während des Animpfprozesses werden die Stammzellen in mehreren Wiederholungen aufgewirbelt und haben dazwischen jeweils Zeit sich auf Trägermaterialien zu setzen und zu adhärieren. Mit jeder Wiederholung steigt das Signal (Kapazität und Permeativität), da die Sonde z.B. einen Bereich von ca. 30mm Radius um die Sonde misst und mit jedem Wiederholungsschritt mehr Zellen in diesem Messbereich verbleiben. Nach dem letzten Wiederholungsschritt erreicht das Signal einen konstanten Wert und ein Plateau. Dieser Wert gibt dann die Anfangskonzentration der Zellen im Reaktor für den folgenden Expansionsprozess vor.

### Expansionsprozess

Im Verlauf des Expansionsprozesses nimmt die Zellzahl der Stammzellen exponentiell zu. Dieses Wachstum ist mittels der Impedanzsonde beobachtbar und die Signalinterpretation gut umsetzbar, da die Signale linear mit der Zellzahl korrelieren. Weiterhin wird offline oder online eine Glukosekonzentrationsbestimmung durchgeführt mittels welcher die ermittelten Zellkonzentrationen bestätigt werden. Diese Information bestätigt dann den jeweilig notwendigen Mediumswechsel. Ist die gewünschte Zellzahl erreicht, so wird über die Prozesssteuerung der Ernteprozess eingeleitet.

### Ernteprozess

Ist der Ernteprozess nach der Expansion in z.B. dem Festbettreaktor eingeleitet, so ändert sich das Signal während des Einwirkens des Enzyms, da sich die Zellgröße beim Ablösen der Zellen von dem Trägermaterial ändert, ebenso wie sich die Membraneigenschaften ändern. Werden die Zellen ausgeströmt, so sinkt das Signal während des Prozesses, bis ein relativ konstanter Wert erreicht wird. Parallel dazu steigt das Signal im Auffanggefäß für die geernteten Zellen, bis ein Plateau erreicht wird. Dieses signalisiert den Zeitpunkt zum Abbruch des Ernteverfahrens.

### Differenzierungsprozess

Werden Stammzellen differenziert, so verändern sich auch ihre Membraneigenschaften. Dieser Differenzierungsprozess wird durch Impedanzspektroskopie beobachtet und gemessen.

### Abkürzungsliste der Abbildungen

(R1) Festbettreaktor
(R2) Konditionierungsgefäß
(P1) Pumpe
(P2) Pumpe
(S3) Messort
(M1) Membranmodul
(S1) Kontrolleinheit
(S2) Kontrolleinheit
(S3) Kontrolleinheit
(S4) Kontrolleinheit
(S5) Kontrolleinheit
(V.1) Ventil
(V.2) Ventil
(V.3) Ventil
(V.4) Ventil
(V.5) Ventil
(V.6) Ventil
(V.7) Ventil
(V.8) Ventil
(V.9) Ventil
(W2) Waage
(G1) Gefäß
(G2) Gefäß
(G3) Gefäß
(G4) Gefäß
(G5) Gefäß
(D1) Doppelmantel
   Figur 1 zeigt eine schematische Darstellung der erfindungsgemäßen Vorrichtung
   Figur 2 zeigt eine schematische Darstellung des erfindungsgemäßen Verfahrens

## Patentansprüche

1. Vorrichtung zur Expansion, Ernte und Differenzierung von Stammzellen umfassend
einen Bioreaktor mit einem Festbettreaktor (R1) mit einem daran gekoppelten Konditionierungsgefäß (R2), wobei Festbettreaktor (R1) und das Konditionierungsgefäß (R2) über Leitungen mit Pumpen (P1, P2) gekoppelt sind und eine Prozesssteuerung vorhanden ist, die Messwerte von Kontrolleinheiten (S1, S2, S3), die mit einer Impedanzsonde erzeugt werden, entgegen nimmt und daraufhin Ventile (V1, V2, V3, V4, V4, V6, V7, V8, V9) steuert, sodass dem Festbettreaktor (R1) aus Gefäßen (G1, G2, G3, G4) Nährstoffe zugeführt werden und die Expansion, Ernte und Differenzierung der Stammzellen automatisch und steril erfolgt.

2. Verfahren zur Expansion, Ernte und Differenzierung von Stammzellen in einem Bioreaktor gemäß Anspruch 1, **gekennzeichnet dadurch, dass** es die Schritte umfasst:
a) Bereitstellen eines Bioreaktor mit geeignetem Medium, animpfen mit einer Starterkultur aus Stammzellen
b) Messung der Sauerstoff-, Glukose- und/oder Laktatkonzentration, der Zelldichte, der Zellzahl, der Zellgrößenverteilung im Bioreaktor durch Impedanzspektroskopie
c) Vergleich der Messwerte aus Schritt b) mit Referenzwerten
d) Automatische Zufuhr von Nährstoffen oder Ernte der Stammzellen durch Entleerung des Bioreaktors und Auffangen der Stammzellen
e) Differenzierung der Stammzellen.

## Claims

1. Device for the expansion, harvesting and differentiation of stem cells, comprising a bioreactor having a fixed-bed reactor (R1) with a conditioning vessel (R2) coupled thereto, the fixed-bed reactor (R1) and the conditioning vessel (R2) being coupled to pumps (P1, P2) by means of pipes, and a process control being provided which receives, from monitoring units (S1, S2, S3), measured data that have been generated with an impedance probe and in response thereto controls valves (V1, V2, V3, V4, V4, V6, V7, V8, V9), so that nutrients are supplied to the fixed-bed reactor (R1) from vessels (G1, G2, G3, G4) and the expansion, harvesting and differentiation of the stem cells take place automatically and in sterile manner.

2. Method for the expansion, harvesting and differentiation of stem cells in a bioreactor according to claim 1, **characterised in that** it comprises the following steps:
a) providing a bioreactor with suitable medium, inoculating with a starter culture of stem cells
b) measuring the oxygen, glucose and/or lactate concentration, the cell density, the cell count and the cell size distribution in the bioreactor by impedance spectroscopy
c) comparing the measured data from step b) with reference data
d) automatically supplying nutrients or harvesting the stem cells by emptying the bioreactor and collecting the stem cells
e) differentiating the stem cells.

## Revendications

1. Dispositif servant à l'expansion, à la récolte et à la différenciation de cellules souches, comprenant un bioréacteur doté d'un réacteur à lit fixe (R1) couplé à un récipient de conditionnement (R2), où le réacteur à lit fixe (R1) et le récipient de conditionnement (R2) sont couplés à des pompes (P1, P2) par l'intermédiaire de conduites et où un système de commande de processus reçoit des valeurs de mesure d'unités de contrôle (S1, S2, S3) générées à l'aide d'une sonde d'impédance puis commande des soupapes (V1, V2, V3, V4, V4, V6, V7, V8, V9) de sorte que des substances nutritives sont amenées au réacteur à lit fixe (R1) depuis des récipients (G1, G2, G3, G4) et que l'expansion, la récolte et la différenciation des cellules souches sont effectuées de manière automatique et stérile.

2. Procédé servant à l'expansion, à la récolte et à la différenciation de cellules souches dans un bioréacteur selon la revendication 1, **caractérisé en ce qu'**il comprend les étapes suivantes :
a) fournir un bioréacteur avec un milieu adapté, procéder à un dopage avec une culture starter constituée de cellules souches ;
b) mesurer la concentration d'oxygène, de glucose et de lactate, la densité de cellules, le nombre de cellules, la distribution de tailles de cellules dans le bioréacteur par une spectroscopie d'impédance ;
c) comparer les valeurs de mesure obtenues à l'étape b) aux valeurs de référence ;
d) amener de manière automatique des substances nutritives ou récolter des cellules souches en vidant le bioréacteur, et collecter les cellules souches ;
d) différencier les cellules souches.
